# EUROPEAN PATENT SPECIFICATION

(11) **EP 3 446 676 B1**
(45) Date of publication and mention of the grant of the patent: **16.09.2020**
(21) Application number: 16899513.2
(22) Date of filing: 20.04.2016
(51) Int. Cl.: A61K 8/37, A61K 8/33, A61K 8/49, A61K 8/368, A61K 8/34, A61Q 13/00, A61K 8/31, A61Q 19/00, A61K 8/9789

(54) **AROMA COMPOSITION**
AROMAZUSAMMENSETZUNG
COMPOSITION AROMATIQUE

(43) Date of publication of application: 27.02.2019
(73) Proprietor: LG Household & Health Care Ltd., Seoul 03184 (KR)
(72) Inventor: KANG, Sunghwi, Daejeon 34114 (KR); YUN, Bo Im, Daejeon 34114 (KR)
(74) Representative: Goddar, Heinz J.
(86) International application number: PCT/KR2016/004092
(87) International publication number: WO 2017/183744

(56) References cited:
- WO-A1-97/31094
- WO-A1-2004/082652
- WO-A1-2015/165582
- CN-A- 102 643 718
- KR-A- 20090 051 607
- KR-A- 20120 053 095
- KR-A- 20120 109 021
- KR-A- 20150 128 191
- KR-A- 20160 097 767

## Description

### [Technical Field]

The specification relates to an aroma composition which can reproduce the *Matthiola incana* fragrance.

### [Background Art]

*Matthiola incana* is a plant included in the family Cruciferae (Brassicaceae). It is also commonly called stock.

*Matthiola incana,* which is its scientific name, is naturalized on the Mediterranean coast and grows to a height of 30 to 60 cm. It is native to the European region. Its stem is as hard as a tree and has white hairs, and its leaves are slanted, ash green-colored, and lanceolate. The leaves have rounded margins and whitish hairs.

Flowers are cross-shaped and bloom from the early spring of April to May. The flowers have a variety of colors and are pleasantly scented.

Branches are divided or undivided, and the undivided branches make divisions by cutting sprouts. Double-flowered plants are sterile, but plants in single-flowered and double-flowered forms can be produced from seeds of single-flowered plants. The plants are weak in cold weather and treated as annuals.

In France, when a man met an ideal type of woman, he used to put this flower in a hat by way of a promise, "I will never have an affair." This plant is the birth flower of May 6 and July 16, and means "eternal beauty" in the language of flowers. In addition, this plant means a strong person who can positively overcome any difficulty and the way it is now is the best.

*Matthiola incana* has an original fragrance but is less popular than other flowers, and thus it is required to develop an aroma composition which is differentiated by reproducing the scent of *Matthiola incana* and has new characteristics.

### [Patent Literature]

Korean Patent Application Publication No. 2004-0031023

### [Disclosure]

### [Technical Problem]

The present invention provides an aroma composition which reproduces the original fragrance of *Matthiola incana* and has high preferability.

### [Technical Solution]

According to an exemplary embodiment of the present invention, the present invention provides an aroma composition which includes: a *Matthiola incana* fragrance ingredient; and an artificial aroma ingredient, wherein the artificial aroma ingredient includes one or two or more selected from the group consisting of hedione, ethylene brassylate and benzyl salicylate.

In addition, according to an exemplary embodiment of the present invention, the present invention provides a preparation for topical use, which includes the aroma composition.

According to an exemplary embodiment of the present invention, the present invention provides a personal care product which includes the aroma composition.

According to an exemplary embodiment of the present invention, the present invention provides a home care product including the aroma composition.

### [Advantageous Effects]

An aroma composition according to an exemplary embodiment of the present invention can reproduce the *Matthiola incana* fragrance.

In addition, the aroma composition according to an exemplary embodiment of the present invention has high similarity and/or high satisfaction with the *Matthiola incana* fragrance.

### [Description of Drawings]

Hereinafter, the specification will be described in further detail.

In the specification, when one part "includes" a component, unless particularly mentioned otherwise, another component may be further included, not excluding other components.

Throughout the specification, the "combination thereof' included in Markush-type expressions refers to a mixture or combination of one or more selected from the group consisting of components described by a Markush-type expression, and one or more selected from the group consisting of the components.

According to an exemplary embodiment of the specification, an aroma composition includes a *Matthiola incana* fragrance ingredient; and an artificial aroma ingredient, wherein the artificial aroma ingredient includes one or two or more selected from the group consisting of hedione, ethylene brassylate and benzyl salicylate.

The *Matthiola incana* fragrance ingredient according to an exemplary embodiment of the specification is a natural ingredient of *Matthiola incana.*

According to an exemplary embodiment of the specification, the *Matthiola incana* fragrance ingredient includes eugenol, methyl eugenol, D-limonene, anethole, phenethyl alcohol, phenyl acetaldehyde and isoeugenol.

According to another exemplary embodiment of the specification, the *Matthiola incana* fragrance ingredient further includes one or two or more selected from the group consisting of benzyl acetate, methyl salicylate and anisaldehyde.

The eugenol, methyl eugenol, D-limonene, anethole, phenethyl alcohol, phenyl acetaldehyde, isoeugenol, benzyl acetate, methyl salicylate and anisaldehyde are main ingredients of the *Matthiola incana* fragrance. However, with only the *Matthiola incana* fragrance ingredients, the original fragrance of *Matthiola incana* may not be reproduced.

Since the aroma composition according to an exemplary embodiment of the specification may further include one or two or more artificial aroma ingredients selected from the group consisting of hedione, ethylene brassylate and benzyl salicylate in addition to the *Matthiola incana* fragrance ingredient, it is possible to reproduce the *Matthiola incana* fragrance, and have high preferability.

The *Matthiola incana* fragrance ingredient may be obtained by a solid phase microextraction (SPME) method which is effective in analysis of a fragrance material of a natural substance.

Generally, to obtain a fragrance, extraction with a solvent or distillation with steam is used, but the above-mentioned methods results in the loss or change of a fragrance ingredient by heating or vacuum pressure during a process of extracting the fragrance ingredient, and thus the composition of the specification has many differences in fragrance characteristics from the original material. As a method of compensating these disadvantages, a headspace method or a solid phase microextraction (SPME) method was developed. Since these two methods, unlike a conventional method, need neither a solvent nor pretreatment of a sample, a large amount of sample is not needed, and since a fragrance ingredient can be analyzed with a sample itself, these methods may be very good methods to reproduce an original fragrance included in the sample.

According to an exemplary embodiment of the present invention, the *Matthiola incana* fragrance ingredient may be analyzed and/or obtained by the SPME method, but the present invention is not limited thereto.

According to an exemplary embodiment of the present invention, a content of the *Matthiola incana* fragrance ingredient is 85 to 98 wt% based on the total weight of the aroma composition, and a content of the artificial aroma ingredient is 2 to 15 wt% based on the total weight of the aroma composition.

When the content of the artificial aroma ingredient is less than 2 wt% based on the total aroma composition, the sense of the floral scent of *Matthiola incana* may be insufficient, and when the content of the artificial aroma ingredient is more than 15 wt%, an animal scent is generated, which may cause a problem. Therefore, within the above-mentioned range, the aroma composition may have high reproducibility of the fragrance of *Matthiola incana* and/or high preferability. However, the content described above is not limited, and may vary according to the purpose and formulation of the composition.

According to an exemplary embodiment of the present invention, the artificial aroma ingredient includes one or two or more selected from the group consisting of hedione, ethylene brassylate and benzyl salicylate.

According to an exemplary embodiment of the present invention, the artificial aroma ingredient includes hedione. Specifically, the artificial aroma ingredient includes one or two or more selected from the group consisting of hedione, ethylene brassylate, and benzyl salicylate.

According to another exemplary embodiment of the present invention, the artificial aroma ingredient includes hedione, ethylene brassylate and benzyl salicylate.

The hedione, ethylene brassylate and benzyl salicylate are artificial aroma ingredients with a gorgeous white floral scent and a delicate and soft musk scent. While they are not present in naturally-occurring *Matthiola incana,* when they are used in addition to the major fragrance ingredient of *Matthiola incana,* the composition exhibits a scent similar to the characteristic original fragrance of *Matthiola incana.* In the specification, particularly, the use and content of hedione are factors most highly affecting the original fragrance of *Matthiola incana.*

According to an exemplary embodiment of the present invention, the *Matthiola incana* fragrance ingredient includes eugenol, methyl eugenol, D-limonene, anethole, phenethyl alcohol, phenyl acetaldehyde and isoeugenol, and the artificial aroma ingredient includes hedione, ethylene brassylate and benzyl salicylate.

Based on the total weight of the aroma composition, the eugenol content is 20 to 35 wt%, the methyl eugenol content is 15 to 25 wt%, the D-limonene content is 10 to 18 wt%, the anethole content is 5 to 10 wt%, the phenethyl alcohol content is 1 to 5 wt%, the phenyl acetaldehyde content is 0.5 to 4 wt%, the isoeugenol content is 0.3 to 3 wt%, the hedione content is 1 to 7 wt%, the ethylene brassylate content is 0.2 to 4 wt%, and the benzyl salicylate content is 0.5 to 5 wt%.

According to another exemplary embodiment of the specification, the *Matthiola incana* fragrance ingredient further includes benzyl acetate, methyl salicylate and anisaldehyde, and based on the total weight of the aroma composition, the benzyl acetate content is 0.3 to 2 wt%, the methyl salicylate content is 0.1 to 2.5 wt%, and the anisaldehyde content is 0.005 to 1.0 wt%.

In the specification, the *Matthiola incana* fragrance ingredient and the artificial aroma ingredient may be a commercially available product, or a product produced by isolation from the plants or synthesis, but the present invention is not limited thereto.

According to another exemplary embodiment of the present invention, a preparation for topical use, which includes the aroma composition, is provided.

The preparation for topical use may be a basic cosmetic, a makeup cosmetic, a body cosmetic or an ointment, and there is no particular limitation to its formulation and thus the preparation may be one selected from the group consisting of a gel, a lotion, an ampoule, an emulsion, an ointment, a patch, a spray or a cream.

A formulation of the basic or body cosmetic may be a lotion, a cream, an essence, a gel, a tonic, a pack, a nourishing cream, a massage cream, an emulsion, a body cleanser, a sunscreen cream or a sunscreen oil, but the present invention is not limited thereto.

In addition, a formulation of the makeup cosmetic may be a foundation, a makeup base, a lipstick, an eye shadow, an eye liner, a mascara, or an eyebrow pencil, but the present invention is not limited thereto.

According to an exemplary embodiment of the present invention, a personal care product which includes the aroma composition is provided.

The personal care product may be a hair product such as a shampoo, a rinse, a conditioner, a wax, a gel or a mousse; an oral product such as a toothpaste or a mouthwash; a skin cleanser such as a soap, a cleansing cream, a cleansing foam, a cleansing water or a cleansing oil; or a perfume, but the present invention is not limited thereto.

According to an exemplary embodiment of the present invention, a home care product including the aroma composition is provided.

The home care product may be an air freshener; or a detergent such as a liquid detergent, a dishwashing soap, a laundry detergent, a bathroom detergent, but the present invention is not limited thereto.

In addition, according to another exemplary embodiment of the present invention, the composition may further include an additive conventionally used in the art, such as a flavoring, a pigment, an antiseptic, an antioxidant, a preservative, a moisturizer, a thickening agent, an inorganic salt or a synthetic polymer depending on a formulation.

According to the formulation and purpose, a content of the active ingredient may vary. For example, a wash-off type cosmetic such as a makeup remover or a detergent, which has an active ingredient remaining on the skin for a short period of time, may be contained at a relatively high concentration. However, a leave-on type cosmetic such as a tonic, an emulsion, a cream or an essence, which has an active ingredient remaining on the skin for a long period of time, may be contained at lower concentrations than the wash-off type cosmetics.

The preparation for topical use, the personal care product and the home care product may be included without departing from the purpose of the specification, the content and formulation thereof are not limited, and a desired formulation may be prepared by selecting the above-described additive as needed.

### [Examples]

Hereinafter, the specification will be described in detail with reference to examples to help in understanding the specification. However, examples according to the specification may be modified in a variety of different forms, and it should be construed that the scope of the specification is not limited to the following examples. The examples of the specification are provided to more completely explain the specification to those of ordinary skill in the art.

### Experimental Example 1: Analysis of Matthiola incana fragrance ingredient using SPME method

An SPME method is a method for obtaining a fragrance ingredient of a wild-type sample, and was used in this experimental example, and ingredient analysis was performed using a method described in a literature as follows.

Approximately two bundles of *Matthiola incana* were wrapped with a Tedlar bag, and a needle for SPME connected with an adsorbent-coating fine glass fiber was inserted thereinto. For three hours after the needle for SPME was inserted thereinto, a fragrance ingredient was captured. The fragrance ingredient-capturing needle for SPME was connected to a gas chromatography instrument to analyze the type and content of the captured fragrance ingredient, and the result is shown in Table 1 below.

### <Analysis conditions>

Analysis instrument: Agilent 6890 NGC/5975 MSD
Detector: MSD
Column: DB-WAX 60 m x250 µ×0.25 µm
Inlet temperature: 230°C
Detecting part temperature: 230°C
Oven temperature: 50°C (5 min) to 230°C (20 min), 3.0°C/min
Split Ratio: 20: 1
Mobile phase gas: He
Injection amount: 0.5 µl

**[Table 1]**

| Name of Aroma Ingredient | wt% |
|---|---|
| Eugenol | 33.71 |
| Methyl eugenol | 23.07 |
| D-limonene | 11.34 |
| Anethole | 8.03 |
| Phenethyl alcohol | 4.02 |
| Phenyl acetaldehyde | 1.34 |
| Isoeugenol | 0.89 |
| Benzyl acetate | 0.63 |
| Methyl salicylate | 0.18 |
| Para-anisaldehyde | 0.09 |
| Others | 16.7 |
| Total content | 100 |

According to the result of the analysis, it was confirmed that the fragrance ingredients of naturally-occurring *Matthiola incana* include 33.71 wt% of eugenol, 23.07 wt% of methyl eugenol, 11.34 wt% of D-limonene, 8.03 wt% of anethole, 4.02 wt% of phenethyl alcohol, 1.34 wt% of phenyl acetaldehyde, 0.89 wt% of isoeugenol, 0.63 wt% of benzyl acetate, 0.18 wt% of methyl salicylate and 0.09 wt% of anisaldehyde. In addition, it was confirmed that a total weight of the ingredients was approximately 83.3 wt% with respect to the entire naturally-occurring *Matthiola incana* fragrance ingredients.

### Experimental Example 2: Preparation of aroma composition

*Matthiola incana* has a characteristic unique combination of a sweet, spicy and floral scent and a fresh and gorgeous white floral scent. According to the composition of Table 2 below, in addition to the *Matthiola incana* fragrance ingredients, the artificial aroma ingredients including hedione, ethylene brassylate and benzyl salicylate, which exhibit the above-described characteristic of the *Matthiola incana* fragrance, were added, thereby preparing aroma compositions of Comparative Example 1 and Examples 1 to 5.

**[Table 2]**

| Name of Aroma Ingredient | Comparative Example1 | Example 1 | Example2 | Example3 | Example4 | Example5 |
|---|---|---|---|---|---|---|
| Eugenol | 33.71 | 30.50 | 27.84 | 24.23 | 22.50 | 21.10 |
| Methyl eugenol | 23.07 | 23.75 | 20.54 | 18.46 | 17.45 | 15.67 |
| D-limonene | 11.34 | 13.84 | 14.21 | 16.76 | 16.87 | 17.82 |
| Anethole | 8.03 | 8.75 | 7.98 | 6.23 | 6.23 | 5.45 |
| Phenethyl alcohol | 4.02 | 4.02 | 4.37 | 4.54 | 4.54 | 4.73 |
| Phenyl acetaldehyde | 1.34 | 1.75 | 2.34 | 2.58 | 2.99 | 3.43 |
| Isoeugenol | 0.89 | 0.53 | 0.40 | 0.40 | 0.35 | 0.35 |
| Benzyl acetate | 0.63 | 0.63 | 1.05 | 1.24 | 1.59 | 1.87 |
| Methyl salicylate | 0.18 | 0.36 | 0.24 | 0.68 | 1.21 | 2.16 |
| Anisaldehyde | 0.09 | 0.09 | 0.10 | 0.09 | 0.08 | 0.05 |
| Hedione | - | - | - | 6.70 | 3.47 | 6.86 |
| Ethylene brassylate | - | - | 2.40 | 2.11 | 2.17 | 2.12 |
| Benzyl salicylate | - | - | 3.37 | 2.23 | 3.77 | 4.41 |
| Dipropyleneglycol | 16.7 | 15.78 | 15.16 | 13.75 | 16.78 | 13.98 |
| Total content | 100.0 | 100.0 | 100.0 | 100.0 | 100.0 | 100.0 |

| | | | | | | |
|---|---|---|---|---|---|---|
| (Units: wt%) | | | | | | |

### Experimental Example 3: Evaluation of fragrance reproducibility of aroma composition

Sensory evaluation for similarity to the *Matthiola incana* fragrance and preferability was performed on 25 healthy males and females aged 27 to 50. The average age of the evaluation subjects was 32.8 years old, and the subjects included 2 males and 23 females.

Reproducibility of the *Matthiola incana* original fragrance was evaluated for the aroma compositions prepared in Comparative Example 1 and Examples 1 to 5 using a 5-point scoring system as shown in Table 3 below by filling in a questionnaire.

An evaluation room and a sample preparation room were separated so that the evaluators were not aware of the sample in advance.

Results of evaluating the reproducibility of the *Matthiola incana* original fragrances of Comparative Example 1 and Examples 1 to 5 are shown in Table 4 below.

**[Table 3]**

| Score | Similarity |
|---|---|
| 1 | Very different |
| 2 | Slightly different |
| 3 | So-so |
| 4 | Slightly similar |
| 5 | Very similar |

**[Table 4]**

| No. | Age | Sex | Comparative Example 1 | Example 1 | Example 2 | Example 3 | Example 4 | Example 5 |
|---|---|---|---|---|---|---|---|---|
| 1 | 27 | Female | 1 | 2 | 2 | 4 | 4 | 4 |
| 2 | 38 | Female | 2 | 3 | 3 | 4 | 4 | 5 |
| 3 | 28 | Female | 1 | 2 | 1 | 4 | 4 | 4 |
| 4 | 39 | Female | 1 | 1 | 2 | 5 | 3 | 5 |
| 5 | 36 | Female | 1 | 2 | 2 | 4 | 3 | 4 |
| 6 | 24 | Female | 1 | 1 | 2 | 5 | 4 | 5 |
| 7 | 36 | Female | 2 | 1 | 3 | 4 | 4 | 4 |
| 8 | 37 | Female | 2 | 1 | 2 | 4 | 3 | 4 |
| 9 | 29 | Female | 1 | 2 | 2 | 3 | 4 | 4 |
| 10 | 33 | Female | 1 | 2 | 1 | 4 | 3 | 4 |
| 11 | 33 | Female | 2 | 1 | 3 | 4 | 4 | 5 |
| 12 | 34 | Female | 2 | 2 | 2 | 3 | 3 | 3 |
| 13 | 33 | Female | 1 | 1 | 2 | 3 | 3 | 4 |
| 14 | 36 | Female | 2 | 2 | 3 | 4 | 3 | 3 |
| 15 | 27 | Female | 2 | 2 | 3 | 4 | 4 | 4 |
| 16 | 38 | Female | 1 | 2 | 2 | 4 | 4 | 4 |
| 17 | 31 | Female | 2 | 3 | 2 | 5 | 4 | 4 |
| 18 | 38 | Female | 1 | 1 | 3 | 4 | 4 | 5 |
| 19 | 32 | Female | 1 | 2 | 2 | 3 | 4 | 4 |
| 20 | 37 | Female | 1 | 2 | 3 | 4 | 3 | 4 |
| 21 | 27 | Male | 1 | 1 | 2 | 4 | 4 | 5 |
| 22 | 27 | Female | 1 | 2 | 1 | 4 | 3 | 4 |
| 23 | 32 | Female | 1 | 2 | 2 | 5 | 3 | 5 |
| 24 | 39 | Male | 2 | 1 | 3 | 5 | 3 | 4 |
| 25 | 29 | Female | 1 | 1 | 2 | 4 | 4 | 4 |
| Average | 32.8 | | 1.36 | 1.68 | 2.2 | 4.04 | 3.56 | 4.2 |

Referring to Table 5, while Comparative Example 1 was prepared using the major ingredients of the fragrance of the naturally-occurring *Matthiola incana,* analyzed in Experimental Example 1, and their contents, very low similarity was exhibited. While Example 1 had the same ingredients as Comparative Example 1 but at various contents, the similarity of the fragrance was slightly increased.

However, Examples 2 to 5 including one or two or more artificial aroma ingredients selected from hedione, ethylene brassylate and benzyl salicylate were evaluated as having increased similarity with the *Matthiola incana* original fragrance.

Particularly, it was confirmed that similarity varies according to the use and content of hedione, and when the artificial aroma ingredients such as hedione, ethylene brassylate and benzyl salicylate are used together, similarity is increased the most.

### Experimental Example 4: Evaluation of satisfaction of fragrance of aroma composition

Sensory evaluation for similarity to a *Matthiola incana* fragrance and preferability was performed on 25 healthy males and females aged 27 to 50. The average age of the evaluation subjects was 32.8 years old, and the subjects included 2 males and 23 females.

The satisfaction of the fragrance of the aroma composition was evaluated for the aroma compositions prepared in Comparative Example 1 and Examples 1 to 5 using a 5-point scoring system as shown in Table 5 below by filling in a questionnaire.

An evaluation room and a sample preparation room were separated so that the evaluators were not aware of the sample in advance.

Results of evaluating the satisfaction of the fragrance of the aroma compositions of Comparative Example 1 and Examples 1 to 5 are shown in Table 6 below.

**[Table 5]**

| Score | Satisfaction |
|---|---|
| 1 | Very dissatisfied |
| 2 | Slightly dissatisfied |
| 3 | So-so |
| 4 | Slightly satisfied |
| 5 | Very satisfied |

**[Table 6]**

| No. | Age | Sex | Comparative Example 1 | Example 1 | Example 2 | Example 3 | Example 4 | Example 5 |
|---|---|---|---|---|---|---|---|---|
| 1 | 27 | Female | 1 | 2 | 3 | 5 | 4 | 4 |
| 2 | 38 | Female | 2 | 3 | 3 | 4 | 4 | 5 |
| 3 | 28 | Female | 2 | 3 | 3 | 3 | 4 | 4 |
| 4 | 39 | Female | 1 | 1 | 2 | 5 | 3 | 5 |
| 5 | 36 | Female | 2 | 2 | 2 | 4 | 3 | 4 |
| 6 | 24 | Female | 1 | 2 | 2 | 5 | 4 | 5 |
| 7 | 36 | Female | 2 | 1 | 3 | 5 | 4 | 4 |
| 8 | 37 | Female | 2 | 3 | 2 | 4 | 3 | 5 |
| 9 | 29 | Female | 1 | 2 | 2 | 3 | 4 | 4 |
| 10 | 33 | Female | 1 | 2 | 1 | 4 | 3 | 4 |
| 11 | 33 | Female | 2 | 1 | 3 | 3 | 4 | 5 |
| 12 | 34 | Female | 2 | 2 | 2 | 3 | 3 | 3 |
| 13 | 33 | Female | 1 | 2 | 2 | 5 | 4 | 4 |
| 14 | 36 | Female | 2 | 2 | 3 | 4 | 3 | 3 |
| 15 | 27 | Female | 2 | 2 | 3 | 4 | 4 | 4 |
| 16 | 38 | Female | 1 | 2 | 2 | 4 | 4 | 4 |
| 17 | 31 | Female | 3 | 3 | 2 | 5 | 5 | 4 |
| 18 | 38 | Female | 1 | 1 | 3 | 4 | 4 | 5 |
| 19 | 32 | Female | 1 | 2 | 2 | 4 | 4 | 4 |
| 20 | 37 | Female | 2 | 2 | 3 | 3 | 3 | 4 |
| 21 | 27 | Male | 1 | 3 | 2 | 4 | 4 | 5 |
| 22 | 27 | Female | 2 | 2 | 2 | 4 | 3 | 4 |
| 23 | 32 | Female | 1 | 3 | 2 | 3 | 4 | 5 |
| 24 | 39 | Male | 2 | 2 | 3 | 4 | 3 | 5 |
| 25 | 29 | Female | 1 | 2 | 2 | 4 | 4 | 4 |
| Average | 32.8 | | 1.56 | 2.08 | 2.36 | 4 | 3.68 | 4.28 |

Referring to Table 6, while Comparative Example 1 was prepared using the major ingredients of the fragrance of the naturally-occurring *Matthiola incana,* synthesized in Experimental Example 1, and their contents, very low satisfaction was exhibited. While Example 1 had the same ingredients as Comparative Example 1 at various contents, Example 1 also exhibited satisfaction lower than the average.

However, Examples 2 to 5 including one or two or more artificial aroma ingredients selected from hedione, ethylene brassylate and benzyl salicylate were evaluated as having increased satisfaction. Particularly, it was confirmed that satisfaction varies according to the use and content of hedione, and when the artificial aroma ingredients such as hedione, ethylene brassylate and benzyl salicylate are used together, satisfaction is increased the most.

Referring to Tables 4 and 6, it was confirmed that, when artificial aroma ingredients of one or two or more selected from the group consisting of hedione, ethylene brassylate and benzyl salicylate are included in addition to the *Matthiola incana* fragrance ingredient, high similarity and satisfaction with the *Matthiola incana* original fragrance are improved.

## Claims

1. An aroma composition, comprising:
*a Matthiola incana* fragrance ingredient; and
an artificial aroma ingredient,
wherein the artificial aroma ingredient includes one or two or more selected from the group consisting of hedione, ethylene brassylate and benzyl salicylate.

2. The composition according to claim 1, wherein the *Matthiola incana* fragrance ingredient includes eugenol, methyl eugenol, D-limonene, anethole, phenethyl alcohol, phenyl acetaldehyde and isoeugenol.

3. The composition according to claim 1, further comprising:
one or two or more selected from the group consisting of benzyl acetate, methyl salicylate and anisaldehyde.

4. The composition according to claim 1, wherein the artificial aroma ingredient includes hedione.

5. The composition according to claim 1, wherein a content of the *Matthiola incana* fragrance ingredient is 85 to 98 wt% based on the total weight of the aroma composition, and
a content of the artificial aroma ingredient is 2 to 15 wt% based on the total weight of the aroma composition.

6. The composition according to claim 2, wherein the artificial aroma ingredient includes hedione, ethylene brassylate and benzyl salicylate, and
based on the total weight of the aroma composition,
the eugenol content is 20 to 35 wt%, the methyl eugenol content is 15 to 25 wt%, the D-limonene content is 10 to 18 wt%, the anethole content is 5 to 10 wt%, the phenethyl alcohol content is 1 to 5 wt%, the phenyl acetaldehyde content is 0.5 to 4 wt%, the isoeugenol content is 0.3 to 3 wt%, the hedione content is 1 to 7 wt%, the ethylene brassylate content is 0.2 to 4 wt%, and the benzyl salicylate content is 0.5 to 5 wt%.

7. The composition according to claim 6, wherein the *Matthiola incana* fragrance ingredient further includes benzyl acetate, methyl salicylate and anisaldehyde, and
based on the total weight of the aroma composition,
the benzyl acetate content is 0.3 to 2 wt%,
the methyl salicylate content is 0.1 to 2.5 wt%, and
the anisaldehyde content is 0.005 to 1.0 wt%.

8. A preparation for topical use, comprising:
the aroma composition according to any one of claims 1 to 7.

9. A personal care product, comprising:
the aroma composition according to any one of claims 1 to 7.

10. A home care product, comprising:
the aroma composition according to any one of claims 1 to 7.

## Patentansprüche

1. Aromazusammensetzung, welche umfasst:
einen *Matthiola incana*-Duftstoffbestandteil; und
einen künstliches Aroma-Bestandteil,
wobei der künstliches Aroma-Bestandteil einen oder mehrere einschließt, ausgewählt aus der Gruppe bestehend aus Hedion, Ethylenbrassylat und Benzylsalicylat.

2. Zusammensetzung nach Anspruch 1, wobei der *Matthiola incana-*Duftstoffbestandteil Eugenol, Methyleugenol, D-Limonen, Anethol, Phenethylalkohol, Phenylacetaldehyd und Isoeugenol einschließt.

3. Zusammensetzung nach Anspruch 1, weiter umfassend:
eines oder zwei oder mehrere, ausgewählt aus der Gruppe bestehend aus Benzylacetat, Methylsalicylat und Anisaldehyd.

4. Zusammensetzung nach Anspruch 1, wobei der künstliches Aroma-Bestandteil Hedion einschließt.

5. Zusammensetzung nach Anspruch 1, wobei ein Gehalt des *Matthiola incana-*Duftstoffbestandteils 85 bis 98 Gewichtsprozent, basierend auf dem Gesamtgewicht der Aromazusammensetzung, ist, und
ein Gehalt des künstliches Aroma-Bestandteils 2 bis 15 Gewichtsprozent, basierend auf dem Gesamtgewicht der Aromazusammensetzung, ist.

6. Zusammensetzung nach Anspruch 2, wobei der künstliches Aroma-Bestandteil Hedion, Ethylenbrassylat und Benzylsalicylat einschließt, und
basierend auf dem Gesamtgewicht der Aromazusammensetzung,
der Eugenolgehalt 20 bis 35 Gewichtsprozent ist, der Methyleugenolgehalt 15 bis 25 Gewichtsprozent ist, der D-Limonengehalt 10 bis 18 Gewichtsprozent ist, der Anetholegehalt 5 bis 10 Gewichtsprozent ist, der Phenethylalkoholgehalt 1 bis 5 Gewichtsprozent ist, der Phenylacetaldehydgehalt 0,5 bis 4 Gewichtsprozent ist, der Isoeugenolgehalt 0,3 bis 3 Gewichtsprozent ist, der Hediongehalt 1 bis 7 Gewichtsprozent ist, der Ethylenbrassylatgehalt 0,2 bis 4 Gewichtsprozent ist und der Benzylsalicylatgehalt 0,5 bis 5 Gewichtsprozent ist.

7. Zusammensetzung nach Anspruch 6, wobei der *Matthiola incana-*Duftstoffbestandteil ferner Benzylacetat, Methylsalicylat und Anisaldehyd einschließt, und
basierend auf dem Gesamtgewicht der Aromazusammensetzung,
der Benzylacetatgehalt 0,3 bis 2 Gewichtsprozent ist,
der Methylsalicylatgehalt 0,1 bis 2,5 Gewichtsprozent ist, und
der Anisaldehydgehalt 0,005 bis 1,0 Gwichtsprozent ist.

8. Zubereitung zur topischen Verwendung, umfassend:
die Aromazusammensetzung nach einem der Ansprüche 1 bis 7.

9. Körperpflegeprodukt, umfassend:
die Aromazusammsensetzung nach einem der Ansprüche 1 bis 7.

10. Hauspflegeprodukt, umfassend:
die Aromazusammensetzung nach einem der Ansprüche 1 bis 7.

## Revendications

1. Composition aromatique comprenant :
un ingrédient parfumant dérivé de *Matthiola incana* ; et
un ingrédient aromatique artificiel,
où l'ingrédient aromatique artificiel inclut un ou deux ou plus sélectionnés dans le groupe consistant en l'hédione, le brassylate d'éthylène et le salicylate de benzyle.

2. Composition selon la revendication 1, où l'ingrédient parfumant dérivé de *Matthiola incana* inclut l'eugénol, le méthyleugénol, le D-limonène, l'anéthole, l'alcool phénéthylique, l'aldéhyde phénylacétique et l'isoeugénol.

3. Composition selon la revendication 1 comprenant en outre :
un ou deux ou plus sélectionnés dans le groupe consistant en l'acétate de benzyle, le salicylate de méthyle et l'anisaldéhyde.

4. Composition selon la revendication 1, où l'ingrédient aromatique artificiel inclut l'hédione.

5. Composition selon la revendication 1, où une teneur en l'ingrédient parfumant dérivé de *Matthiola incana,* rapportée au poids total de la composition aromatique, va de 85 à 98 % en poids et
une teneur en l'ingrédient aromatique artificiel, rapportée au poids total de la composition aromatique, va de 2 à 15 % en poids.

6. Composition selon la revendication 2, où l'ingrédient aromatique artificiel inclut l'hédione, le brassylate d'éthylène et le salicylate de benzyle et,
rapportées au poids total de la composition aromatique,
la teneur en eugénol va de 20 à 35 % en poids, la teneur en méthyleugénol va de 15 à 25 % en poids, la teneur en D-limonène va de 10 à 18 % en poids, la teneur en anéthole va de 5 à 10 % en poids, la teneur en alcool phénéthylique va de 1 à 5 % en poids, la teneur en aldéhyde phénylacétique va de 0,5 à 4 % en poids, la teneur en isoeugénol va de 0,3 à 3 % en poids, la teneur en hédione va de 1 à 7 % en poids, la teneur en brassylate d'éthylène va de 0,2 à 4 % en poids et la teneur en salicylate de benzyle va de 0,5 à 5 % en poids.

7. Composition selon la revendication 6, où l'ingrédient parfumant dérivé de *Matthiola incana* inclut en outre l'acétate de benzyle, le salicylate de méthyle et l'anisaldéhyde et,
rapportées au poids total de la composition aromatique,
la teneur en acétate de benzyle va de 0,3 à 2 % en poids,
la teneur en salicylate de méthyle va de 0,1 à 2,5 % en poids et
la teneur en anisaldéhyde va de 0,005 à 1,0 % en poids.

8. Préparation à usage topique comprenant :
la composition aromatique selon l'une quelconque des revendications 1 à 7.

9. Produit d'hygiène personnelle comprenant :
la composition aromatique selon l'une quelconque des revendications 1 à 7.

10. Produit d'entretien ménager comprenant :
la composition aromatique selon l'une quelconque des revendications 1 à 7.
